# EUROPEAN PATENT APPLICATION

(11) **EP 3 884 944 A1**
(43) Date of publication of application: **29.09.2021**
(21) Application number: 19887759.9
(22) Date of filing: 18.11.2019
(51) Int. Cl.: A61K 31/4406, A61P 35/00

(54) **APPLICATION OF CHIDAMIDE**

(30) Priority: 20.11.2018 CN 201811385440
(71) Applicant: Shenzhen Chipscreen Biosciences Co., Ltd., Shenzhen, Guangdong 518057 (CN)
(72) Inventor: LU, Xianping, Shenzhen, Guangdong 518057 (CN); HUANG, Huiqiang, Shenzhen, Guangdong 518057 (CN); LI, Wenyu, Shenzhen, Guangdong 518057 (CN); FU, Xin, Shenzhen, Guangdong 518057 (CN)
(74) Representative: Colombo, Stefano Paolo
(86) International application number: PCT/CN2019/119094
(87) International publication number: WO 2020/103778

(57) **Abstract**

The present invention relates to the technical field of medicine, and discloses an application of Chidamide. The present invention provides the application of a therapeutic schedule for using Chidamide in the treatment of B cell lymphoma, and verifies by clinical test the outstanding effect of Chidamide monotherapy for diffuse large B cell lymphoma and recurrent or refractory follicular lymphoma accompanied by specific epigenetic regulation gene mutation. The application can treat B cell lymphoma patients more effectively.

## Description

The present application claims the priority of the Chinese patent application that was filed with the Chinese Patent Office on November 20, 2018, and has the application number of 201811385440.8 and the invention title of "Use of Chidamide", and whose entire content is incorporated in the present application by reference.

### Technical Field

The present invention relates to the technical field of medicine, in particular to use of Chidamide.

### Background Art

B-cell lymphoma mainly includes diffuse large B-cell lymphoma (DLBCL), follicular lymphoma (FL), marginal zone B-cell lymphoma (MZL), mantle cell lymphoma (MCL) and so on. At present, the R-CHOP regimen of rituximab (R) combined with cyclophosphamide (CTX), adriamycin (ADR), vincristine (VCR) and prednisone (Pred) is used as standard first-line treatment regimen for diffuse large B cell lymphoma (DLBCL), and has achieved good long-term survival. Under the current conventional immunochemotherapy, 1/3 of patients still have no response to treatment or relapse, and there is still room for improvement in efficacy, such as changing the combination of conventional chemotherapies or adding targeted drugs. High-risk elderly DLBCL patients have poor efficacy for R-CHOP, with a CR rate of only about 70% and poor long-term survival, and the efficacy needs to be improved urgently.

At the same time, for relapsed and refractory patients, chemotherapy is still the main means of rescue therapy. The commonly used regimens for second-line treatment include regimens that have non-cross resistance with the first-line regimen, less effect on hematopoiesis, and no effect on succeeding collection of stem cells, such as Dice, ICE and GEMOX regimens, for rescue therapy, but there still is a considerable part of patients who have no improvement in disease treatment. According to literature reports, for the patients with relapsed or refractory B cell lymphoma who received DICE regimen for re-induction, the overall efficiency was about 50% to 60%, and there was a considerable number of patients unable to obtain improvement in disease treatment, so the exploration of more efficient rescue regimens is currently in need for the patients with relapsed or refractory B cell lymphoma.

### Contents of the Invention

In view of this, it is an object of the present invention to provide use of Chidamide in the manufacture of a medicament for treating B-cell lymphoma and/or in the treatment of B-cell lymphoma. In the specific embodiments for carrying out the present invention, clinical trials were performed on a plurality of relapsed or refractory B cell lymphomas such as follicular lymphoma (FL), marginal zone B-cell lymphoma (MALT), lymphoplasmacytic lymphoma (LPL), small lymphocyte B cell lymphoma (SLL), diffuse large B cell lymphoma (DLBCL) as specific diseases to be treated.

Chidamide (Epidaza) is a subtype selective histone deacetylase (HDAC) inhibitor independently researched and developed in China, and is a new drug of class 1.1. The use of Chidamide for its first indication, monotherapy for relapsed or refractory peripheral T-cell lymphoma (PTCL), was approved by the China Food and Drug Administration (CFDA) on December 23, 2014, and thus it is the first oral subtype selective HDAC inhibitor for this indication in the world approved for marketing. Chidamide mainly targets the subtypes 1, 2 and 3 of class I and the subtype 10 of class IIb of HDAC, and has a regulatory effect on abnormal epigenetic functions of tumors. It induces chromatin remodeling by inhibiting related HDAC subtypes to increase the acetylation level of chromatin histone, and thus leads to alterations in gene expression of multiple signaling pathways (i.e., epigenetic alterations), thereby inhibiting tumor cell cycle and inducing tumor cell apoptosis, and having overall regulatory activity on the body's cellular immunity, and inducing and enhancing the tumor killing effect mediated by natural killer cells (NK) and antigen-specific cytotoxic T cells (CTL). Chidamide also has functions such as inducing tumor stem cell differentiation and reversing the epithelial-mesenchymal phenotype transition (EMT) of tumor cells through epigenetic regulation mechanisms, thereby playing a potential role in restoring the sensitivity of drug-resistant tumor cells to drugs and inhibiting tumor metastasis and recurrence, etc.

The results of the phase I clinical trial of Chidamide showed that the effective remission rate of Chidamide in monotherapy of T-cell non-Hodgkin's malignant lymphoma was 80%, but for the 3 cases of B-cell non-Hodgkin's lymphoma patients as enrolled, one case showed progression of disease after treatment with Chidamide, and the other two showed stable disease without curative effect, so the above results indicated that Chidamide alone showed no effectiveness in the treatment of B-cell lymphoma.

However, it is unexpectedly found in the present invention that Chidamide monotherapy is effective in the treatment of B cell lymphoma, especially relapsed or refractory lymphoma, especially diffuse large B cell lymphoma, and relapsed or refractory follicular lymphoma accompanied with specific epigenetic regulatory gene mutation; therefore, preferably, the B cell lymphoma is relapsed or refractory follicular lymphoma accompanied with specific epigenetic regulatory gene mutation and/or diffuse large B cell lymphoma.

At the same time, the present invention also provides a preparation for treating B-cell lymphoma, which uses Chidamide as a main active ingredient, and is added with other active ingredients and/or preparation auxiliary materials that do not affect each other. The other active ingredients that do not affect each other may be an active ingredient for treating B cell lymphoma, or may be an active ingredient for treating other diseases, or a combination of the two.

Further, the present invention also provides a method for treating B cell lymphoma, which comprises administering an effective dose of Chidamide.

It is known from the above technical solution that the present invention proposes use of a therapeutic regimen of administering Chidamide with therapeutic effect on B cell lymphoma, and verifies with clinical trials that the monotherapy of Chidamide has a more prominent effect in the treatment of diffuse large B cell lymphoma and relapsed or refractory follicular lymphoma accompanied with specific epigenetic regulatory gene mutation, and the use can treat patients with B cell lymphoma more efficiently.

### Specific Embodiments for Carrying Out the Invention

The present invention discloses use of Chidamide, and those skilled in the art can learn from the content herein and appropriately improve the process parameters to achieve the same. It should be particularly pointed out that all similar substitutions and modifications are obvious to those skilled in the art, and they are all deemed to be included in the present invention. The use of the present invention has been described through the preferred examples, and those skilled in the art can obviously make changes or appropriate alterations and combinations to the use described herein without departing from the content, spirit and scope of the present invention so as to implement and apply the technology of the present invention.

The following is a further description of the use of Chidamide provided by the present invention.

### Example 1: Phase II clinical trial of Chidamide monotherapy in the treatment of relapsed or refractory B cell lymphoma

**Test drugs:** Chidamide tablets: off-white tablets, 5 mg/tablet. Produced by Shenzhen Chipscreen Biosciences Co., Ltd.
**Dosage regimen:** 2 times a week, 30 mg each time, the interval of two administrations should not be less than 3 days (for example, on Monday and Thursday, Tuesday and Friday, Wednesday and Saturday, etc.). The administration was performed 30 minutes after breakfast, every 3 weeks was a treatment cycle. During the entire study, all subjects should unceasingly receive the treatment under study until the appearance of any one of the following conditions: progression of disease, intolerable adverse reaction, death, withdrawal from treatment, withdrawal of the informed consent or loss to follow-up.
**Number of cases:** Simon's optimal two-stage design was adopted, 72 patients were enrolled, and pathological subtypes included: follicular lymphoma (FL) grade 1, grade 2 or grade 3, marginal zone B-cell lymphoma (MALT), lymphoplasmacytic lymphoma (LPL), small lymphocyte B cell lymphoma (SLL), diffuse large B cell lymphoma (DLBCL).

### Enrollment criteria:

1. According to the WHO 2008 diagnosis criteria, patients who were histopathologically diagnosed as B cell lymphoma, including diffuse large B cell lymphoma (DLBCL), follicular lymphoma (FL) grade I, grade II or grade III, marginal zone B cell lymphoma (MALT), lymphoplasmacytic lymphoma (LPL), small lymphocyte B cell lymphoma (SLL), mantle cell lymphoma (MCL), transformed lymphoma (TL);
2. Patients who were previously sensitive to cytotoxic drug regimens
   (Note: the definition of "sensitive": disease remission, efficacy evaluated as PR or CR (confirmed or not confirmed); disease relapsed 6 months after remission);
3. Patients with DLBCL, FL grade 3, MALT, LPL, SLL subtypes who had previously received at least 2 chemotherapy regimens;
Patients with FL grade 1 or grade 2 who had previously received at least 3 chemotherapy regimens;
Note: The first-line therapeutic regimens should comprise a combined chemotherapy of anthracycline, such as CHOP; high-dose chemotherapy with self-stem cell transplantation support was deemed as one therapeutic regimen; for the treatment combinations or drugs that were defined as new therapy, the change from CVP to CHOP was deemed as new therapy, while it was not deemed as new therapy when the same therapy or drug treatment was used again;
4. There was at least one measurable lesion, and its longest diameter should be greater than 1.5cm, or its short diameter should be greater than 1.0 cm;
5. Age: 18 to 75 years old;
6. ECOG score: 0 to 2;
7. Expected survival: ≥3 months;
8. The main organ functions were in accordance with the following criteria within 7 days before treatment:
   (1) Blood routine examination criteria (under state without blood transfusion in 14 days):
      Hemoglobin (HB): ≥ 80g/L;
      Absolute neutrophil count (ANC): ≥ 1.5×10⁹/L;
      Platelet (PLT): ≥ 60×10⁹/L;
   (2) Biochemical examination should meet the following criteria:
      Total bilirubin (TBIL): ≤1.5 times upper limit of normal value (ULN);
      Alanine aminotransferase (ALT) and aspartate aminotransferase AST: ≤ 2.5× ULN, if accompanied with liver metastasis, ALT and AST: ≤ 5× ULN
      Serum creatinine (Cr) ≤1.5× ULN or creatinine clearance rate (CCr): ≥ 60 ml/min;
   9. Cardiac Doppler ultrasound evaluation: left ventricular ejection fraction (LVEF): ≥ lower limit of normal value (50%);
   10. Males and females in childbearing age who agreed to adopt reliable contraceptive means during the study and within 4 weeks after the end of treatment under study;
   11. Patient who voluntarily participated in this study and signed an informed consent.

### Therapeutic regimen:

The patients were orally administrated with Chidamide according to the aforementioned dosage regimen, and the safety- and efficacy evaluation were performed as required at the specified time.
1. Safety evaluation
   (1) Blood routine examination was performed per week;
   (2) Physical examination was performed every 3 weeks, and the vital signs and ECOG scores were recorded;
   (3) Blood biochemical examination was performed every 3 weeks, including:
      Liver functions: alanine aminotransferase (ALT), aspartate aminotransferase (AST), total bilirubin (TBIL), direct bilirubin (DBIL), glutamyl transpeptidase (GGT), albumin (ALB)
      Renal functions: urea nitrogen (BUN), creatinine (Cr)
      Fasting blood glucose
      Electrolytes: potassium, sodium, chlorine, calcium, magnesium
      Lactate dehydrogenase (LDH)

Other safety examination was performed once every 6 weeks, including:
Urinary routine examination
12-leads electrocardiogram (simultaneously calculating QTC)
2. Efficacy evaluation
Efficacy evaluation time: efficacy evaluation was performed once every 6 weeks.
Efficacy evaluation means: the evaluation of lymph nodes and organ lesions was performed by the same imaging method as the baseline (enhanced CT of cervicothoracic/abdominal pelvis, PET/CT, nuclear magnetic resonance, X-ray chest film, abdominal ultrasonography, etc.) and physical examination method.
Efficacy evaluation criteria: the evaluation was performed by referring to the International Working Group Criteria for Efficacy Evaluation of Non-Hodgkin's Lymphoma (IWC).
Follow-up period after treatment

All subjects were followed up after the treatment, and the follow-up time was started at the end of the treatment, and lasted for 2 years after the treatment under study for the last case subject was completed.

In the first year after treatment, the follow-up was performed once every 3 months. In the second year after treatment, the follow-up was performed every 6 months.

**Clinical trial results:** 10 cases were enrolled, and 8 cases had been evaluated, in which the ORR was 37.5%, and the benefit rate was 62.5%.

The results showed that Chidamide monotherapy is effective in the treatment of B cell lymphoma.

### Example 2: Multicenter, single-arm, open clinical trial of Chidamide tablet in the treatment of relapsed or refractory follicular lymphoma (FL) accompanied with specific epigenetic regulatory gene mutation

**Test drugs:** Chidamide tablets: off-white tablets, 5 mg/tablet. Produced by Shenzhen Chipscreen Biosciences Co., Ltd.
**Dosage regimen:** 2 times a week, 30 mg each time, the interval of two administrations should not be less than 3 days (for example, on Monday and Thursday, Tuesday and Friday, Wednesday and Saturday, etc.). The administration was performed 30 minutes after breakfast, every 3 weeks was a treatment cycle. During the entire study, all subjects should unceasingly receive the treatment under study until the appearance of any one of the following conditions: progression of disease, intolerable adverse reaction, death, withdrawal from treatment, withdrawal of the informed consent or loss to follow-up.
**Number of cases:** 33 patients were enrolled in this clinical trial, including 10 cases enrolled in the first phase.

### Enrollment criteria:

1. According to the WHO 2008 diagnosis criteria, patients who were histopathologically diagnosed as follicular lymphoma (FL) grade 1, grade 2 or grade 3a;
2. Patients who had received at least one systematic treatment (including rituximab-containing regimen, hematopoietic stem cell transplantation) but showed no remission or relapsed after remission;
3. Having at least one of the following conditions: involvement lymph node regions: ≥3, diameter for each ≥ 3cm; any lymph node or extranodal tumor: ≥ 7cm; appearance of B symptom; hypersplenotrophy; pleural effusion or ascites; hypocytosis (white cells <1.0×10⁹/L, platelet <100×10⁹/L); leukemia;
4. Having CREBBP and/or EP300 specific genetic mutation that was confirmed by second-generation sequencing;
5. Having at least one evaluable lesion;
6. Age: between 18 to 75 years old, male or female;
7. ECOG physical score: 0 to 1;
8. Absolute neutrophil count: ≥ 1.5×10⁹/L, platelet: ≥ 80×10⁹/L, hemoglobin: ≥ 90g/L;
9. Expected survival: ≥ 3 months;
10. Not receiving therapy such as radiotherapy, chemotherapy, targeted therapy or hematopoietic stem cell transplantation within the first 4 weeks before being enrolled;
11. Voluntarily signing informed consent in written form.

### Research steps:

This clinical trial comprised screening period, treatment period, and follow-up period.

### 1. Screening period

After obtaining the informed consent, the patients were screened by history collection, physical examination, laboratory examination, tumor assessment, and the first genetic test samples were collected and detected during the screening period (baseline samples);

Qualified patients with CREBBP and/or EP300 mutations entered the trial after screening.

### 2. Treatment period

Patients were orally administrated with Chidamide according to the regimen, and the following safety- and efficacy follow-ups were performed at the specified times:
(1) Safety follow-ups
   ① Blood routine examination was performed once every week;
   ② Physical examination was performed once every 6 weeks, and vital signs and ECOG scores were recorded;
   ③ Blood biochemical examination was performed once every 6 weeks, including:
      Liver functions: alanine aminotransferase (ALT), aspartate aminotransferase (AST), total bilirubin (TBIL), direct bilirubin (DBIL), glutamyl transpeptidase (GGT), albumin (ALB)
      Renal functions: urea nitrogen (BUN), creatinine (CR)
      Fasting blood glucose;
      Electrolytes: potassium, sodium, chlorine, calcium, magnesium
      Lactate dehydrogenase (LDH)
   ④ Other safety examinations were performed once every 6 weeks, including:
      Urinary routine examination
      12-leads electrocardiogram (simultaneously calculating QTC)
(2) Efficacy follow-ups
   Follow-up interval: Efficacy assessment was carried out once every 6 weeks; and efficacy follow-up was performed at the end of treatment.
   Efficacy evaluation means: Evaluation of lymph nodes and organ lesions, skin lesions was performed by imaging methods (CT or PET/CT), clinical examination, bone marrow puncture and biopsy. The imaging methods used for patients must be the same at all follow-up points.
   Efficacy evaluation criteria: For those of CT scanning, the evaluation was performed according to the 1999 International Working Group Criteria for Efficacy Evaluation of Non-Hodgkin's Lymphoma (IWG); for those of PET/CT scanning, the evaluation was performed according to the 2007 International Coordination Program's Revised Guideline Criteria (Appendix 1).
   Gene detection sample collection: If the efficacy evaluation during treatment showed disease progression (PD), the second genetic test samples collection and detection were performed (PD sample). The residual swollen lesions or metabolic sites with high FDG uptake collected from the patients at PD period were sampled and subjected to biopsy again.

### 3. Follow-up period

All subjects were followed up after the treatment was completed, and the follow-up time was started at the end of treatment, and lasted for 2 years, until the subjects showed tumor progression or death. Telephone follow-up was allowed.

In the first year after treatment, the follow-up was performed once every 3 months. In the second year after treatment, the followed-up was performed once every 6 months.

### Efficacy indicators:

### (1) Main efficacy indicators

Objective remission rate (ORR), including cases and rates of complete remission (CR), complete remission unconfirmed (CRu), and partial remission (PR)

### (2) Secondary efficacy indicators

Disease control rate (DCR), including cases and rates of complete remission (CR), complete remission unconfirmed (CRu), partial remission (PR), and stable disease (SD);
Progression-free survival (PFS);
Overall survival (OS);
Correlation analysis of efficacy and specific mutation;

**Clinical trial results:** In the pre-test, a total of 11 patients accompanied with epigenetic mutation B-NHL were observed, the ORR was 72.7%, and the CR was 36.3%.

The results show that Chidamide has better efficacy in the treatment of relapsed or refractory follicular lymphoma (FL) accompanied with specific epigenetic regulatory gene mutation.

The above are only the preferred embodiments of the present invention, and it should be pointed out for those of ordinary skill in the art, without departing from the principle of the present invention, several improvements and modifications can be made and these improvements and modifications should also be regarded as falling into the scope sought to be protected by the present invention.

## Claims

1. Use of Chidamide in the manufacture of a medicament for treating B-cell lymphoma and/or in the treatment of B-cell lymphoma.

2. The use according to claim 1, wherein the B-cell lymphoma is relapsed or refractory B-cell lymphoma.

3. The use according to claim 1 or 2, wherein the B-cell lymphoma is diffuse large B-cell lymphoma.

4. The use according to claim 1 or 2, wherein the B-cell lymphoma is relapsed or refractory follicular lymphoma accompanied with specific epigenetic regulatory gene mutation.

5. A preparation for treating B-cell lymphoma, which is **characterized by** using Chidamide as a main active ingredient, and being added with other active ingredients and/or preparation auxiliary materials that do not affect each other.

6. A method for treating B-cell lymphoma, which is **characterized by** administering an effective dose of Chidamide.
